# EUROPEAN PATENT SPECIFICATION

(11) **EP 4 093 272 B1**
(45) Date of publication and mention of the grant of the patent: **05.03.2025**
(21) Application number: 21744390.2
(22) Date of filing: 21.01.2021
(51) Int. Cl.: A61B 5/0215, A61B 5/00, A61F 2/24, A61B 17/11, A61B 17/00

(54) **INTRACARDIAC PRESSURE SENSOR WITH BARRIER STRUCTURE AND ASSOCIATED SYSTEMS**
INTRAKARDIALER DRUCKSENSOR MIT SPERRSTRUKTUR, ZUGEHÖRIGE SYSTEME
CAPTEUR DE PRESSION INTRACARDIAQUE AVEC STRUCTURE DE BARRIÈRE ET SYSTÈMES ASSOCIÉS

(30) Priority: 21.01.2020 US 202062963686 P
(43) Date of publication of application: 30.11.2022
(60) Divisional application: 24223188.4
(73) Proprietor: Shifamed Holdings, LLC, Campbell, CA 95008 (US)
(72) Inventor: FAHEY, Brian, Campbell, California 95008 (US); ANDRIOLA, Peter, Campbell, California 95008 (US); SCHRECK, Stefan, Campbell, California 95008 (US)
(74) Representative: Forresters IP LLP
(86) International application number: PCT/US2021/014433
(87) International publication number: WO 2021/150765

(56) References cited:
- WO-A1-2019/142152
- WO-A1-2019/142152
- WO-A1-2019/186101
- US-A1- 2007 060 959
- US-A1- 2007 118 039
- US-A1- 2010 063 375
- US-A1- 2013 144 379
- US-A1- 2017 105 635
- US-A1- 2017 105 635

## Description

### CROSS-REFERENCE TO RELATED APPLICATION(S)

This application claims the benefit of U.S. Provisional Patent Application No. 62/963,686, filed January 21, 2020.

### TECHNICAL FIELD

The present technology generally relates to implantable medical devices and, in particular, to implantable intracardiac sensors and associated systems and methods for monitoring physiological parameters of a patient's heart.

### BACKGROUND

Heart failure is a medical condition associated with the inability of the heart to effectively pump blood to the body. Heart failure affects millions of people worldwide, and may arise from multiple root causes, but is generally associated with myocardial stiffening, myocardial shape remodeling, and/or abnormal cardiovascular dynamics. Chronic heart failure is a progressive disease that worsens considerably over time. Initially, the body's autonomic nervous system adapts to heart failure by altering the sympathetic and parasympathetic balance. While these adaptations are helpful in the short-term, over a longer period of time they serve to make the disease worse.

Heart failure is a medical term that includes both heart failure with reduced ejection fraction (HFrEF) and heart failure with preserved ejection fraction (HFpEF). The prognosis with both HFpEF and HFrEF is poor; one-year mortality is 26% and 22%, respectively, according to one epidemiology study. In spite of the high prevalence of HFpEF, there remain limited options for HFpEF patients. Pharmacological therapies have been shown to impact mortality in HFrEF patients, but there are no similarly-effective evidence-based pharmacotherapies for treating HFpEF patients. Current practice is to manage and support patients while their health continues to decline.

A common symptom among heart failure patients is elevated left atrial pressure. In the past, clinicians have treated patients with elevated left atrial pressure by creating a shunt between the left and right atria using a blade or balloon septostomy. The shunt decompresses the left atrium (LA) by relieving pressure to the right atrium (RA) and systemic veins. Over time, however, the shunt typically will close or reduce in size. More recently, percutaneous interatrial shunt devices have been developed which have been shown to effectively reduce left atrial pressure. However, these percutaneous devices often have an annular passage with a fixed diameter which fails to account for a patient's changing physiology and condition. For this reason, existing percutaneous shunt devices may have a diminishing clinical effect after a period of time. Many existing percutaneous shunt devices typically are also only available in a single size that may work well for one patient but not another. Also, sometimes the amount of shunting created during the initial procedure is later determined to be less than optimal months later. US 2007/118039 discloses a system and method for the intra corporal, telemetric measuring of blood pressure, particularly within the heart or a great vessel, includes a substantially rigid sensor chip mounted in a holder and an antenna. WO2019/142152 discloses a device for providing a passage between a first and second heart chamber is provided. US 2017/105635 discloses an implantable medical device includes a housing having a proximal end and a distal end, a control module enclosed by the housing, and a pressure sensor electrically coupled to the control module. A fixation member is coupled to the housing distal end for anchoring the housing distal end at a fixation site within a cardiovascular system of a patient, and the pressure sensor is spaced apart proximally from the fixation member. US 2007/060959 discloses a system and method for administering a therapeutic treatment to the heart includes a pressure sensor positioned in the pulmonary artery, an implantable medical device located remotely from the sensor, and communication means for communicating pressure data from the pressure sensor to the implantable medical device. Accordingly, there is a need for improved devices, systems, and methods for treating heart failure patients, particularly those with elevated left atrial pressure.
In a first aspect of the present invention there is provided a system for monitoring intracardiac pressure of a patient as set forth in the claims.

### BRIEF DESCRIPTION OF THE DRAWINGS

FIG. 1 is a schematic illustration of an interatrial device implanted in a heart and configured in accordance with an embodiment of the present technology.
FIG. 2 is a cross-sectional view of tissue overgrowth on an implanted pressure sensor.
FIG. 3 is a cross-sectional view of an intracardiac pressure monitoring device with a barrier structure configured in accordance with an embodiment of the present technology.
FIG. 4 is a schematic illustration of an intracardiac pressure monitoring device with a barrier structure configured in accordance with another embodiment of the present technology.
FIG. 5 is a schematic illustration of another intracardiac pressure monitoring device with a barrier structure configured in accordance with another embodiment of the present technology.
FIG. 6 is a schematic illustration of an intracardiac pressure monitoring device with an expandable barrier structure configured in accordance with a further embodiment of the present technology.

### DETAILED DESCRIPTION

The present technology is generally directed to devices for monitoring intracardiac pressure. In some embodiments, a device includes a pressure sensor configured to measure pressure within a heart chamber of a patient (e.g., a LA and/or a RA). The device further includes a barrier structure configured to reduce or prevent overgrowth of tissue (e.g., endothelialization or pannus) over the pressure sensor. For example, the barrier structure can reduce or prevent tissue overgrowth by partially or completely surrounding the pressure sensor. Alternatively or in combination, the barrier structure can reduce or prevent tissue overgrowth by spacing the pressure sensor away from tissue of the heart chamber (e.g., septal wall tissue).

In some embodiments, the intracardiac pressure monitoring devices described herein are implemented as part of an interatrial shunting system. The system can include a shunting element implantable into a patient at or adjacent a septal wall. The shunting element can fluidly connect a LA and a RA of the patient to facilitate blood flow therebetween. The system can include an intracardiac pressure monitoring device implantable into a heart chamber of the patient (e.g., the LA or RA). Optionally, the system can include a plurality of intracardiac pressure monitoring devices configured for placement in a plurality of different portions of the patient's heart. For example, a system with two intracardiac pressure monitoring devices can include one device implantable in a heart chamber (e.g., the LA) and the other device implantable in a different heart chamber (e.g., the RA). In some embodiments, the system can adjust the geometry and/or flow rate of the shunting element (e.g., by adjusting the size and/or shape of the shunt lumen) based on pressure measurements generated by the intracardiac pressure monitoring device(s).

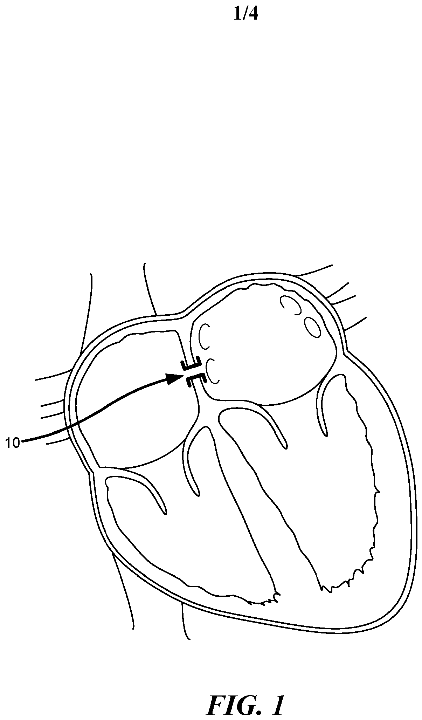

The terminology used in the description presented below is intended to be interpreted in its broadest reasonable manner, even though it is being used in conjunction with a detailed description of certain specific embodiments of the present technology. Certain terms may even be emphasized below; however, any terminology intended to be interpreted in any restricted manner will be overtly and specifically defined as such in this Detailed Description section. Additionally, the present technology can include other embodiments that are within the scope of the examples but are not described in detail with respect to FIGS. 1-6.

Reference throughout this specification to "one embodiment" or "an embodiment" means that a particular feature, structure, or characteristic described in connection with the embodiment is included in at least one embodiment of the present technology. Thus, the appearances of the phrases "in one embodiment" or "in an embodiment" in various places throughout this specification are not necessarily all referring to the same embodiment.

Reference throughout this specification to relative terms such as, for example, "generally," "approximately," and "about" are used herein to mean the stated value plus or minus 10%.

As used herein, the terms "interatrial device," "interatrial shunt device," "IAD," "IASD," "interatrial shunt," and "shunt" are used interchangeably to refer to a device that, in at least one configuration, includes a shunting element that provides a blood flow between a first region (e.g., a LA of a heart) and a second region (e.g., a RA or coronary sinus of the heart) of a patient. Although described in terms of a shunt between the atria, namely the left and right atria, one will appreciate that the technology may be applied equally to devices positioned between other chambers and passages of the heart, or between other parts of the cardiovascular system or other system. For example, any of the shunts described herein, including those referred to as "interatrial," may be nevertheless used and/or modified to shunt between the LA and the coronary sinus, or between the right pulmonary vein and the superior vena cava. Moreover, while the disclosure herein primarily describes shunting blood from the LA to the RA, the present technology can be readily adapted to shunt blood from the RA to the LA to treat certain conditions, such as pulmonary hypertension. For example, mirror images of embodiments, or in some cases identical embodiments, used to shunt blood from the LA to the RA can be used to shunt blood from the RA to the LA in certain patients.

Although certain embodiments herein are described in the context of pressure sensors, one of skill in the art will appreciate that the present technology is equally applicable to sensor for measuring other types of physiologic parameters (e.g., blood flow, blood oxygenation, etc.). Additionally, although certain embodiments herein are discussed as being part of an interatrial shunting system, one of skill in the art will appreciate that the present technology can be implemented in other types of systems for treating heart failure (e.g., systems that do not involve interatrial shunts), or can be used as a standalone device.

The headings provided herein are for convenience only and do not interpret the scope or meaning of the claimed present technology.

### A. Interatrial Shunts for Treatment of Heart Failure

Heart failure can be classified into one of at least two categories based upon the ejection fraction a patient experiences: (1) HFpEF, historically referred to as diastolic heart failure or (2) HFrEF, historically referred to as systolic heart failure. One definition of HFrEF is a left ventricular ejection fraction lower than 35%-40%. Though related, the underlying pathophysiology and the treatment regimens for each heart failure classification may vary considerably. For example, while there are established pharmaceutical therapies that can help treat the symptoms of HFrEF, and at times slow or reverse the progression of the disease, there are limited available pharmaceutical therapies for HFpEF with only questionable efficacy.

In heart failure patients, abnormal function in the left ventricle (LV) leads to pressure build-up in the LA. This leads directly to higher pressures in the pulmonary venous system, which feeds the LA. Elevated pulmonary venous pressures push fluid out of capillaries and into the lungs. This fluid build-up leads to pulmonary congestion and many of the symptoms of heart failure, including shortness of breath and signs of exertion with even mild physical activity. Risk factors for HF include renal dysfunction, hypertension, hyperlipidemia, diabetes, smoking, obesity, old age, and obstructive sleep apnea. HF patients can have increased stiffness of the LV which causes a decrease in left ventricular relaxation during diastole resulting in increased pressure and inadequate filling of the ventricle. HF patients may also have an increased risk for atrial fibrillation and pulmonary hypertension, and typically have other comorbidities that can complicate treatment options.

Interatrial shunts have recently been proposed as a way to reduce elevated left atrial pressure, and this emerging class of cardiovascular therapeutic interventions has been demonstrated to have significant clinical promise. FIG. 1 shows the conventional placement of a shunt in the septal wall between the LA and RA. Most conventional interatrial shunts (e.g., shunt 10) involve creating a hole or inserting a structure with a lumen into the atrial septal wall, thereby creating a fluid communication pathway between the LA and the RA. As such, elevated left atrial pressure may be partially relieved by unloading the LA into the RA. In early clinical trials, this approach has been shown to improve symptoms of heart failure.

One challenge with many conventional interatrial shunts is determining the most appropriate size and shape of the shunt lumen. A lumen that is too small may not adequately unload the LA and relieve symptoms; a lumen that is too large may overload the RA and rightheart more generally, creating new problems for the patient. Moreover, the relationship between pressure reduction and clinical outcomes and the degree of pressure reduction required for optimized outcomes is still not fully understood, in part because the pathophysiology for HFpEF (and to a lesser extent, HFrEF) is not completely understood. As such, clinicians are forced to take a best guess at selecting the appropriately sized shunt (based on limited clinical evidence) and generally cannot adjust the sizing over time. Worse, clinicians must select the size of the shunt based on general factors (e.g., the size of the patient's anatomical structures, the patient's hemodynamic measurements taken at one snapshot in time, etc.) and/or the design of available devices rather than the individual patient's health and anticipated response. With many such traditional devices, the clinician does not have the ability to adjust or titrate the therapy once the device is implanted, for example, in response to changing patient conditions such as progression of disease. By contrast, interatrial shunting systems configured in accordance with embodiments of the present technology allow a clinician to select the size-perioperatively or post-implant-based on the patient.

A further challenge with conventional interatrial shunts is that the function of the LA (and more generally, the cardiovascular system) can vary depending on a number of factors, for example during exercise, during periods where a patient's medication adherence has slipped, as the patient's disease progresses, or during other periods. Existing conventional shunts are generally static in nature and lack an ability to adapt to patient conditions in such a way to optimize therapy.

Other shortcomings of existing conventional interatrial shunts include: (1) shunts tending to be permanently implanted in the septal wall in a way that complicates or prevents future transseptal access, which may prohibit or complicate additional left-heart procedures that generally would require transseptal access; (2) shunts tending to be fixed and unable to adapt to changing patient conditions, such as progression of disease, and (3) a lack of sensors and/or machine-learning capability that limit the information available from the patient and limit the ability to improve therapy for the patient (or for the larger patient cohort) over time.

### B. Intracardiac Pressure Sensors

The present technology is generally directed to devices for monitoring intracardiac pressures that can be used in combination with an interatrial shunting system or other cardiovascular treatment system or as stand-alone devices. Such devices can include one or more pressure sensors implantable within or proximate to the patient's heart (e.g., within a heart chamber such as the LA or the RA, within the coronary sinus, within a pulmonary artery, etc.). For example, the device can include a first pressure sensor implantable within the LA and a second pressure sensor implantable within the RA. Each pressure sensor can be configured to measure a pressure within the corresponding heart chamber. For example, the first pressure sensor can be configured to measure left atrial pressure and the second pressure sensor can be configured to measure right atrial pressure. Optionally, the pressure sensor can measure a plurality of pressures within the heart chamber at a plurality of different time points, e.g., to monitor changes in pressure over time. The pressure measurements can be used to selectively adjust the interatrial shunting system, as described in detail below.

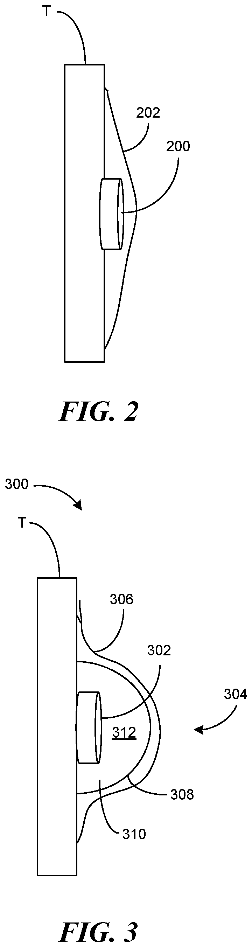

FIG. 2 is a cross-sectional view of tissue overgrowth on an implanted pressure sensor 200. The pressure sensor 200 can be implanted on tissue T of a patient's heart chamber (e.g., a septal wall, a vein, an artery, etc.). In some instances, overgrowth of tissue T surrounding the pressure sensor 200 results in formation of a tissue layer 202 (e.g., pannus or an endothelial layer) that partially or completely covers the pressure sensor 200. The tissue layer 202 can attenuate or otherwise interfere with transmission of pressure from the heart chamber to the pressure sensor 200. As a result, the pressure measurements generated by the pressure sensor 200 can become increasingly inaccurate over time.

In some embodiments, the intracardiac pressure monitoring devices described herein include a barrier structure configured to reduce or prevent tissue overgrowth on an implanted pressure sensor. For example, the barrier structure can partially or completely surround the pressure sensor so as to prevent a tissue layer from forming directly on the surfaces of the pressure sensor. As another example, the barrier structure can space the pressure sensor away from the heart soft tissue such that the pressure sensor does not have any direct contact with the soft tissue after implantation. In such embodiments, the distance between the pressure sensor and the tissue can be sufficiently large to prevent or delay tissue overgrowth from reaching the pressure sensor. The various embodiments described herein are expected to improve the accuracy and performance of implanted pressure sensors, particularly in applications where long-term intracardiac pressure monitoring is desired.

FIG. 3, for example, is a cross-sectional view of an intracardiac pressure monitoring device 300 with a barrier structure 304 configured in accordance with an embodiment of the present technology. The device 300 includes a pressure sensor 302 implanted on tissue T of a patient's heart chamber and configured to measure pressure of the heart chamber. The device 300 further includes a barrier structure 304 coupled to the pressure sensor 302 and configured to reduce or prevent overgrowth of tissue (e.g., tissue layer 306) thereon. In some embodiments, the barrier structure 304 includes a membrane 308 (e.g., a flexible or rigid membrane) coupled to the tissue T in a position surrounding the pressure sensor 302. In the illustrated embodiment, the membrane 308 has a dome-like or hemispherical shape. In other embodiments, however, the membrane 308 can have a different shape (e.g., spherical or semi-spherical, ellipsoidal or semiellipsoidal, cylindrical, etc.).

The membrane 308 can form an enclosed space 310 separated from the blood within the heart chamber. In some embodiments, the enclosed space 310 is filled with a fluid 312, such as a gas or a liquid (e.g., saline, silicon oil, etc.). The pressure sensor 302 can be positioned within the enclosed space 310 formed by the membrane 308, such that the pressure sensor 302 is at least partially surrounded by the fluid 312. As a result, the membrane 308 and fluid 312 can serve as a physical barrier that reduces or prevents tissue overgrowth on the pressure sensor 302. In some embodiments, the membrane 308 and/or fluid 312 are configured to transmit external pressures (e.g., blood pressures within the heart chamber) to the pressure sensor 302 with little or no attenuation (e.g., less than 20%, 10%, 5%, or 1% attenuation). Thus, even though the pressure sensor 302 is enclosed by the membrane 308 and fluid 312, it can still produce accurate measurements of the pressure within the heart chamber. This pressure transmission can be maintained even if there is tissue overgrowth onto the membrane 308, as shown in FIG. 3. Optionally, the membrane 308 can be made of and/or coated with a material that reduces or prevents tissue overgrowth.

For example, in the illustrated embodiment, the membrane 308 forms a dome-like structure having a surface area greater than the surface area of the pressure sensor 302. The attenuation of pressure across the membrane 308 can be approximately proportional to the inverse of the cube of its surface area. As a result, the pressure-attenuating effect of the tissue layer 306 can be reduced. Additionally, the increased surface area of the dome-like structure can increase the blood velocity over the membrane 308, e.g., compared to the blood velocity over an exposed pressure sensor. This increase in blood velocity can further reduce or prevent tissue adhesion to the surface of the membrane 308. The dome-like structure can also protrude further into the heart chamber and/or away from the tissue T than an exposed pressure sensor, which can also prevent or reduce tissue overgrowth.

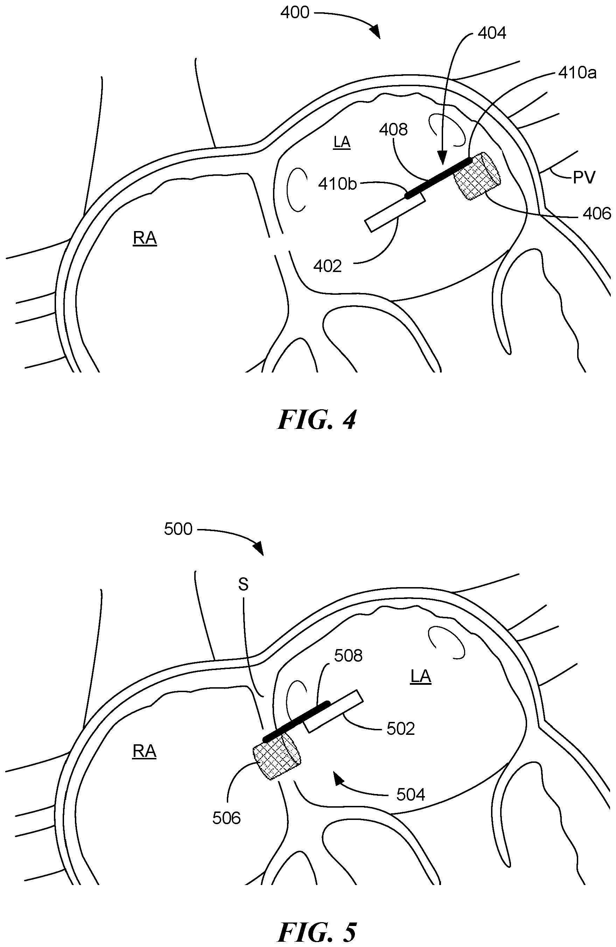

FIG. 4 is a schematic illustration of an intracardiac pressure monitoring device 400 with a barrier structure 404 configured in accordance with another embodiment of the present technology. The device 400 includes a pressure sensor 402 implanted in a patient's heart chamber and configured to measure pressure of the heart chamber. Although FIG. 4 shows the device 400 implanted within the left atrium LA, in other embodiments, the device 400 can be located in a different heart chamber (e.g., the right atrium RA).

The device 400 further includes a barrier structure 404 coupled to the pressure sensor 402 and configured to reduce or prevent overgrowth of tissue thereon. The barrier structure 404 can include an anchoring element 406 coupled to a portion of the heart chamber to secure the pressure sensor 402 in place. The anchoring element 406 can be a stent, basket, cage, hook, barb, or any other structure that can be fastened to a portion of the patient's heart. For example, in the illustrated embodiment, the anchoring element 406 is configured as a stent positioned at least partially within a pulmonary vein PV.

The barrier structure 404 can further include a connecting element 408 coupling the pressure sensor 402 to the anchoring element 406. In some embodiments, the connecting element 408 includes a first end portion 410a coupled to the anchoring element 406 and a second end portion 410b coupled to the pressure sensor 402. The connecting element 408 can be a post, rod, strut, or any other structure having an elongated shape extending away from the inner surface(s) of the heart chamber. As a result, the pressure sensor 402 can be positioned away from the heart tissue when coupled to the connecting element 408. The length of the connecting element 408 can be sufficiently long to reduce or prevent tissue overgrowth onto the pressure sensor 402. For example, the connecting element 408 can have a length of at least 1-2 mm. Optionally, the connecting element 408 can be made of and/or coated with a material that reduces or prevents tissue overgrowth.

FIG. 5 is a schematic illustration of another intracardiac pressure monitoring device 500 with a barrier structure 504 configured in accordance with another embodiment of the present technology. The device 500 can be generally similar to the device 400 described with respect to FIG. 4. Accordingly, like numbers (e.g., barrier structure 504 v. barrier structure 404) are used to identify similar or identical components and discussion of the device 500 illustrated in FIG. 5 will be limited to those features that differ from the embodiment discussed with respect to FIG. 4.

The device 500 includes a pressure sensor 502 coupled to a barrier structure 504. The barrier structure 504 can include an anchoring element 506 coupled at or within the septal wall S between the left atrium LA and the right atrium RA. For example, the septal wall S can include an aperture formed therein (e.g., to receive an interatrial shunting element (not shown)), and the anchoring element 506 can be a stent positioned at least partially within the aperture. Optionally, the anchoring element 506 can be an interatrial shunting element, or a component thereof. The pressure sensor 502 can be coupled to the anchoring element 506 via a connecting element 508. The connecting element 508 can extend away from the anchoring element 506 and into a heart chamber, such that the pressure sensor 502 is positioned within the heart chamber away from the tissue of the septal wall S. As a result, the barrier structure 504 can reduce or prevent tissue overgrowth from the septal wall S onto the pressure sensor 502.

Although FIG. 5 illustrates the pressure sensor 502 as being positioned within the left atrium LA, in other embodiments, the pressure sensor 502 can instead be positioned in a different heart chamber (e.g., the right atrium RA). Optionally, in some embodiments, the device 500 can include a plurality of pressure sensors, such as a first pressure sensor positioned in the left atrium LA and a second pressure sensor positioned in the right atrium RA. In such embodiments, the barrier structure 504 can include a first connecting element extending from the anchoring element 506 into the left atrium LA and a second connecting element extending from the anchoring element 506 into the right atrium RA. The first pressure sensor can be coupled to the first connecting element and the second pressure sensor can be coupled to the second connecting element. As a result, the device 500 can be used to measure pressure in both the left atrium LA and the right atrium RA.

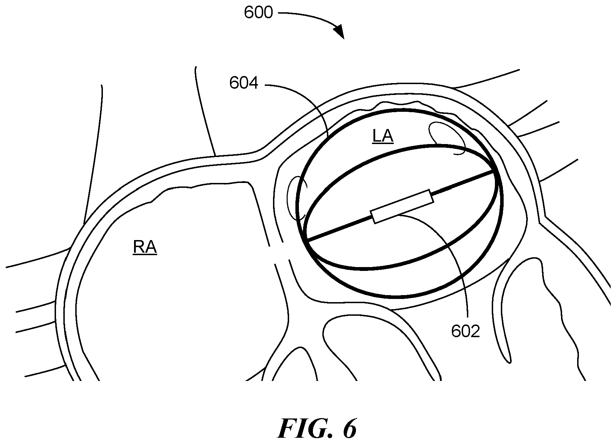

FIG. 6 is a schematic illustration of an intracardiac pressure monitoring device 600 with an expandable barrier structure 604 configured in accordance with a further embodiment of the present technology. The device 600 includes a pressure sensor 602 implanted in a patient's heart chamber and configured to measure pressure of the heart chamber. Although FIG. 6 shows the device 600 implanted within the left atrium LA, in other embodiments, the device 600 can be located in a different heart chamber (e.g., the right atrium RA).

The device 600 further includes an expandable structure 604 serving as a barrier structure for preventing tissue overgrowth onto the pressure sensor 602. The expandable structure 604 can be a cage, basket, mesh, stent, or any other structure that can be deployed from a contracted configuration to an expanded configuration (e.g., as shown in FIG. 6). When in the expanded configuration, the expandable structure 604 can conform to and/or engage the inner surface(s) of the heart chamber, while the pressure sensor 602 can be coupled to a portion of the expandable structure 604 away from the inner surface(s). For example, in the illustrated embodiment, the pressure sensor 602 is suspended within an interior portion of the expandable structure 604. As a result, although some tissue overgrowth onto the expandable structure 602 may occur, the overgrowth is not expected to reach the pressure sensor 602. Optionally, the expandable structure 602 can be made of and/or coated with a material that reduces or prevents tissue overgrowth.

In some embodiments, the intracardiac pressure monitoring devices described herein with respect to FIGS. 3-6 can be implemented as part of an interatrial shunting system. An interatrial shunting system can include a shunting element implantable into a patient at or adjacent a septal wall. The shunting element can fluidly connect a LA and a RA of the patient to facilitate blood flow therebetween. The system can further include one or more intracardiac pressure monitoring devices as described herein with respect to FIGS. 3-6, such as a first device positioned within the LA and a second device positioned with the RA.

In some embodiments, the shunting element of an interatrial shunting system is adjustable (e.g., non-invasively adjustable) to control blood flow between the LA and the RA. For example, the geometry of the shunt lumen (e.g., size and/or shape) can be adjusted using a suitable actuation mechanism coupled to the shunting element. The adjustments can be based on pressure measurements generated by a pressure sensor of an intracardiac pressure monitoring device, as previously described herein. For example, one or more pressure sensors can be used to measure pressure in a heart chamber over time, and adjustments to the shunting element can be made in response to changes in the measured pressure over time (e.g., increasing or decreasing pressure over time). As another example, pressure measurements from one or more pressure sensors can be used to calculate a pressure differential between different heart chambers (e.g., the LA and the RA), and adjustments to the shunting element can be made in response to the calculated pressure differential (e.g., whether the pressure differential is greater than or less than a predetermined threshold, whether the pressure differential exceeds a predetermined range, etc.). Examples of adjustable interatrial shunting systems that can be used with the anchoring mechanisms described herein are described in International Patent Application Nos. PCT/US2020/038549, PCT/US2020/049996, PCT/US/2020/063360, and PCT/ US2020/064529.

As one of skill in the art will appreciate from the disclosure herein, various components of the intracardiac pressure monitoring devices described above can be omitted without deviating from the scope of the present technology. Likewise, additional components not explicitly described above may be added to the intracardiac pressure monitoring devices without deviating from the scope of the present technology. Accordingly, the systems described herein are not limited to those configurations expressly identified, but rather encompasses variations and alterations of the described systems.

### Conclusion

Embodiments of the present disclosure may include some or all of the following components: a battery, supercapacitor, or other suitable power source; a microcontroller, FPGA, ASIC, or other programmable component or system capable of storing and executing software and/or firmware that drives operation of an implant; memory such as RAM or ROM to store data and/or software/firmware associated with an implant and/or its operation; wireless communication hardware such as an antenna system configured to transmit via Bluetooth, WiFi, or other protocols known in the art; energy harvesting means, for example a coil or antenna which is capable of receiving and/or reading an externally-provided signal which may be used to power the device, charge a battery, initiate a reading from a sensor, or for other purposes. Embodiments may also include one or more sensors, such as pressure sensors, impedance sensors, accelerometers, force/strain sensors, temperature sensors, flow sensors, optical sensors, cameras, microphones or other acoustic sensors, ultrasonic sensors, ECG or other cardiac rhythm sensors, SpO2 and other sensors adapted to measure tissue and/or blood gas levels, blood volume sensors, and other sensors known to those who are skilled in the art. Embodiments may include portions that are radiopaque and/or ultrasonically reflective to facilitate image-guided implantation or image guided procedures using techniques such as fluoroscopy, ultrasonography, or other imaging methods. Embodiments of the system may include specialized delivery catheters/systems that are adapted to deliver an implant and/or carry out a procedure. Systems may include components such as guidewires, sheaths, dilators, and multiple delivery catheters. Components may be exchanged via over-the-wire, rapid exchange, combination, or other approaches.

The above detailed description of embodiments of the technology are not intended to be exhaustive or to limit the technology to the precise forms disclosed above. Although specific embodiments of, and examples for, the technology are described above for illustrative purposes, various equivalent modifications are possible within the scope of the technology as those skilled in the relevant art will recognize. For example, although steps are presented in a given order, alternative embodiments may perform steps in a different order. The various embodiments described herein may also be combined to provide further embodiments. For example, although this disclosure has been written to describe devices that are generally described as being used to create a path of fluid communication between the LA and RA, the LV and the right ventricle (RV), or the LA and the coronary sinus, it should be appreciated that similar embodiments could be utilized for shunts between other chambers of heart or for shunts in other regions of the body.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Unless the context clearly requires otherwise, throughout the description and the examples, the words "comprise," "comprising," and the like are to be construed in an inclusive sense, as opposed to an exclusive or exhaustive sense; that is to say, in the sense of "including, but not limited to." As used herein, the terms "connected," "coupled," or any variant thereof, means any connection or coupling, either direct or indirect, between two or more elements; the coupling of connection between the elements can be physical, logical, or a combination thereof. Additionally, the words "herein," "above," "below," and words of similar import, when used in this application, shall refer to this application as a whole and not to any particular portions of this application. Where the context permits, words in the above Detailed Description using the singular or plural number may also include the plural or singular number respectively. As used herein, the phrase "and/or" as in "A and/or B" refers to A alone, B alone, and A and B. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with some embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein. The scope of the present invention is defined by the appended claims.

## Claims

1. A system (300; 400; 500; 600) for monitoring intracardiac pressure of a patient, the system comprising:
a pressure sensor (202; 302; 402; 502; 602) configured to measure a pressure within a heart chamber of the patient;
a shunting element having a lumen extending therethrough, wherein the lumen is configured to fluidly couple a left atrium and a right atrium of the patient when the system is implanted in the patient, wherein the pressure sensor is operably coupled to the shunting element;
an actuation mechanism configured to adjust a geometry of the lumen in response to the measured pressure;
an anchoring element (406; 506) configured to be secured to tissue at and/or proximate the heart chamber; and
a connecting element (408; 508) coupling the pressure sensor to the anchoring element, wherein the connecting element is configured to space the pressure sensor apart from the tissue when the anchoring element is secured to the tissue to reduce tissue overgrowth on the pressure sensor.

2. The system of claim 1 wherein the pressure sensor (202; 302; 402; 502; 602) is configured to be implanted within a left atrium or a right atrium of the patient.

3. The system of claim 1 wherein the anchoring element (406; 506) is configured to be positioned in a septal wall.

4. The system of claim 1 wherein the connecting element (408; 508) is configured to extend at least partially into a left atrium of the patient, and wherein, when implanted, the pressure sensor is suspended within the left atrium and out of contact with heart tissue.

5. The system of claim 1 wherein the actuation mechanism is configured to be selectively adjusted over time based on a plurality of pressure measurements at a plurality of different time points from the pressure sensor.

6. The system of claim 1 wherein the pressure sensor (402; 502) is configured to be positioned spaced apart from tissue such that the pressure sensor is suspended, via the connecting element, within the heart chamber.

7. The system of claim 1 wherein the pressure sensor (202; 302; 402; 502; 602) is configured to measure a plurality of pressures within the heart chamber at a plurality of different time points.

8. The system of claim 1 wherein the pressure sensor is a first pressure sensor configured to be positioned in a left atrium of the patient and measure left atrial pressure, and wherein the system further comprises a second pressure sensor configured to be positioned in a right atrium of the patient and measure right atrial pressure.

9. The system of claim 11 wherein the connecting element is a first connecting element coupling the first pressure sensor to the anchoring element, and wherein the system further comprises a second connecting element coupling the second pressure sensor to the anchoring element.

10. The system of claim 1 wherein the system is further configured to calculate a pressure differential between a left atrium and a right atrium of the patient based on the measured pressure.

11. The system of claim 1 wherein the connecting element has a length of at least 2 mm.

12. The system of claim 1 wherein the connecting element is coated with a material that reduces or prevents tissue overgrowth.

## Patentansprüche

1. System (300; 400; 500; 600) zur Überwachung des intrakardialen Drucks eines Patienten, wobei das System umfasst:
einen Drucksensor (202; 302; 402; 502; 602), der dazu konfiguriert ist, einen Druck innerhalb einer Herzkammer des Patienten zu messen;
ein Shuntelement mit einem Lumen, das sich hindurch erstreckt, wobei das Lumen dazu konfiguriert ist, ein linkes Atrium und ein rechtes Atrium des Patienten fluidisch zu koppeln, wenn das System in den Patienten implantiert ist, wobei der Drucksensor betriebsfähig an das Shuntelement gekoppelt ist;
einen Betätigungsmechanismus, der dazu konfiguriert ist, eine Geometrie des Lumens als Reaktion auf den gemessenen Druck einzustellen;
ein Verankerungselement (406; 506), das dazu konfiguriert ist, an Gewebe an und/oder nahe der Herzkammer gesichert zu sein; und
ein Verbindungselement (408; 508), das den Drucksensor an das Verankerungselement koppelt, wobei das Verbindungselement dazu konfiguriert ist, den Drucksensor von dem Gewebe zu beabstanden, wenn das Verankerungselement an dem Gewebe gesichert ist, um Gewebeüberwachstum auf dem Drucksensor zu reduzieren.

2. System nach Anspruch 1, wobei der Drucksensor (202; 302; 402; 502; 602) dazu konfiguriert ist, in einem linken Atrium oder einem rechten Atrium des Patienten implantiert zu sein.

3. System nach Anspruch 1, wobei das Verankerungselement (406; 506) dazu konfiguriert ist, in einer Septumwand positioniert zu sein.

4. System nach Anspruch 1, wobei das Verbindungselement (408; 508) dazu konfiguriert ist, sich mindestens teilweise in ein linkes Atrium des Patienten zu erstrecken, und wobei, wenn implantiert, der Drucksensor innerhalb des linken Atriums und außer Kontakt mit Herzgewebe suspendiert ist.

5. System nach Anspruch 1, wobei der Betätigungsmechanismus dazu konfiguriert ist, im Laufe der Zeit basierend auf einer Vielzahl von Druckmessungen zu einer Vielzahl von verschiedenen Zeitpunkten von dem Drucksensor ausgehend selektiv eingestellt zu werden.

6. System nach Anspruch 1, wobei der Drucksensor (402; 502) dazu konfiguriert ist, von Gewebe beabstandet positioniert zu sein, so dass der Drucksensor, über das Verbindungselement, innerhalb der Herzkammer suspendiert ist.

7. System nach Anspruch 1, wobei der Drucksensor (202; 302; 402; 502; 602) dazu konfiguriert ist, eine Vielzahl von Drücken innerhalb der Herzkammer zu einer Vielzahl von verschiedenen Zeitpunkten zu messen.

8. System nach Anspruch 1, wobei der Drucksensor ein erster Drucksensor ist, der dazu konfiguriert ist, in einem linken Atrium des Patienten positioniert zu sein und den linken atrialen Druck zu messen, und wobei das System ferner einen zweiten Drucksensor umfasst, der dazu konfiguriert ist, in einem rechten Atrium des Patienten positioniert zu sein und den rechten atrialen Druck zu messen.

9. System nach Anspruch 11, wobei das Verbindungselement ein erstes Verbindungselement ist, das den ersten Drucksensor an das Verankerungselement koppelt, und wobei das System ferner ein zweites Verbindungselement umfasst, das den zweiten Drucksensor an das Verankerungselement koppelt.

10. System nach Anspruch 1, wobei das System ferner dazu konfiguriert ist, ein Druckdifferenzial zwischen einem linken Atrium und einem rechten Atrium des Patienten basierend auf dem gemessenen Druck zu berechnen.

11. System nach Anspruch 1, wobei das Verbindungselement eine Länge von mindestens 2 mm aufweist.

12. System nach Anspruch 1, wobei das Verbindungselement mit einem Material beschichtet ist, das Gewebeüberwachstum reduziert oder verhindert.

## Revendications

1. Système (300 ; 400 ; 500 ; 600) destiné à surveiller la pression intracardiaque d'un patient, le système comprenant :
un capteur de pression (202 ; 302 ; 402 ; 502 ; 602) configuré pour mesurer une pression à l'intérieur d'une cavité cardiaque d'un patient ;
un élément de shuntage comportant un lumen s'étendant à travers celui-ci, dans lequel le lumen est configuré des sorte à fluidiquement relier une oreillette gauche et une oreillette droite du patient lors de l'implantation du système dans le patient, dans lequel le capteur de pression est fonctionnellement relié à l'élément de shuntage ;
un mécanisme d'actionnement configuré pour ajuster une géométrie du lumen en fonction de la pression mesurée ;
un élément d'ancrage (406 ; 506) configuré de sorte à être fixé au tissu et/ou à proximité de la cavité cardiaque ; et
un élément de raccordement (408 ; 508) reliant le capteur de pression à l'élément d'ancrage, dans lequel l'élément de raccordement est configuré pour garder une distance entre le capteur de pression et le tissu lorsque l'élément d'ancrage est fixé au tissu afin de réduire la surcroissance du tissu sur le capteur de pression.

2. Système selon la revendication 1, dans lequel le capteur de pression (202 ; 302 ; 402 ; 502 ; 602) est configuré pour être implanté à l'intérieur d'une oreillette gauche ou d'une oreillette droite du patient.

3. Système selon la revendication 1, dans lequel l'élément d'ancrage (406 ; 506) est configuré pour être positionné dans une paroi septale.

4. Système selon la revendication 1, dans lequel l'élément de raccordement (408 ; 508) est configuré pour s'étendre au moins en partie jusque dans l'oreillette gauche du patient et, dans lequel, une fois implanté, le capteur de pression est suspendu à l'intérieur de l'oreillette gauche et est hors de contact avec le tissu cardiaque.

5. Système selon la revendication 1, dans lequel le mécanisme d'actionnement est configuré pour être sélectivement ajusté au fil du temps en fonction d'une pluralité de mesures de pression du capteur de pression, à une pluralité de moments différents

6. Système selon la revendication 1, dans lequel le capteur de pression (402 ; 502) est configuré pour être positionné à distance du tissu de sorte que le capteur de pression soit suspendu, par l'intermédiaire de l'élément de raccordement, à l'intérieur de la cavité cardiaque.

7. Système selon la revendication 1, dans lequel le capteur de pression (202 ; 302 ; 402 ; 502 ; 602) est configuré pour mesurer une pluralité de pressions à l'intérieur der la cavité cardiaque, à une pluralité de moments différents.

8. Système selon la revendication 1, dans lequel le capteur de pression est un premier capteur de pression configuré pour être positionné dans une oreillette gauche du patient et pour mesurer la pression atriale gauche, et dans lequel le système comprend en outre un second capteur de pression configuré pour être positionné dans une oreillette droite du patient et pour mesurer la pression atriale droite.

9. Système selon la revendication 11, dans lequel l'élément de raccordement est un premier élément de raccordement reliant le premier capteur de pression à l'élément d'ancrage, et dans lequel le système comprend en outre un second élément de raccordement reliant le second capteur de pression à l'élément d'ancrage.

10. Système selon la revendication 1, dans lequel le système est en outre configuré pour calculer un différentiel de pression entre une oreillette gauche et une oreillette droite du patient en fonction de la pression mesurée.

11. Système selon la revendication 1, dans lequel la longueur de l'élément de raccordement est d'au moins 2 mm.

12. Système selon la revendication 1, dans lequel l'élément de raccordement est revêtu d'une matière qui réduit ou prévient la surcroissance du tissu.
